Europäisches Patentamt

European Patent Office

Office européen des brevets

(19)

(11) Veröffentlichungsnummer: **0 275 552 B1**

(12) **EUROPÄISCHE PATENTSCHRIFT**

(45) Veröffentlichungstag der Patentschrift:
07.11.90

(51) Int. Cl.⁵: **A61F 2/60**

(21) Anmeldenummer: 87119299.3

(22) Anmeldetag: 29.12.87

(54) Künstlicher gelenkloser Fuss.

(30) Priorität: 29.12.86 DE 3644612

(43) Veröffentlichungstag der Anmeldung:
27.07.88 Patentblatt 88/30

(45) Bekanntmachung des Hinweises auf die Patenterteilung:
07.11.90 Patentblatt 90/45

(84) Benannte Vertragsstaaten:
AT BE CH DE ES FR GB GR IT LI LU NL SE

(56) Entgegenhaltungen:
EP-A- 0 122 372
US-A- 3 098 239

(73) Patentinhaber: Otto Bock Orthopädische Industrie
Besitz- und Verwaltungs-Kommanditgesellschaft,
Industriestrasse, D-3408 Duderstadt 1(DE)

(72) Erfinder: Haupt, Werner, Friedensstrasse 39,
D-3408 Duderstadt(DE)

(74) Vertreter: Lins, Edgar, Dipl.-Phys. et al, Patentanwälte
Gramm + Lins Theodor-Heuss-Strasse 2,
D-3300 Braunschweig(DE)

**Beschreibung**

Die Erfindung betrifft einen künstlichen gelenklosen Fuß mit einem eine obere Anschlußfläche bildenden inkompressiblen Holzkern und mit elastischen, mit dem Kern verbundenen Kunststoffmaterialien zur Ausbildung der Fußform und Gewährleistung eines flexiblen Abrollvorgangs, wobei der Holzkern auf seiner Anschlußfläche einen dorsalen Anschlag für einen mit ihm verbindbaren Adapter bildet.

Derartige gelenklose Füße sind seit langer Zeit bekannt (s. z.B. EP-A 0 122 372) und werden häufig verwendet. Die gelenklosen Füße lassen sich aufgrund einer geschickten Anordnung von flexiblen Materialien an dem Holzkern so ausbilden, daß sie einem Gelenkfuß im Gehkomfort nicht nachstehen und ihn sogar übertreffen können.

Die Holzkerne derartiger gelenkloser Füße sollten aus möglichst leichtem Holz hergestellt werden. Das leichte und darüber hinaus preiswerte Holz kann an sich alle Funktionen des Kerns eines derartigen gelenklosen Fußes erfüllen, weist jedoch den Nachteil auf, daß an seiner Anschlußfläche im Bereich des dorsalen Anschlages aufgrund seiner Weichheit eine starke Abnutzung einsetzt, die aufgrund der bei jedem Abrollvorgang auftretenden Belastung der Oberfläche durch einen mit dem Fuß verbundenen Metall-Adapter entsteht. Beim Abrollen drückt der auf der Anschlußfläche aufliegende Adapter mit seiner Vorderkante auf die Anschlußfläche des Holzkerns. Bei der Verwendung von leichtem Holz wird die Anschlußfläche an dieser Stelle, also an der Stelle des dorsalen Anschlags, sehr bald deformiert, wodurch die Festigkeit der Verbindung mit dem Adapter nicht mehr gewährleistet ist und unerwünschte Spielbewegungen des Fußes auftreten können. Darüber hinaus kann sogar die Verschraubung des Adapters brechen, so daß die gesamte Fußprothese unbrauchbar und unfallträchtig wird.

Diese Beschädigungsmöglichkeit hat den Einsatz von teurem, harten Holz notwendig gemacht, das allerdings das Gewicht der Fußprothese wesentlich erhöht hat.

Es ist bereits bei internen Versuchen der Anmelderin versucht worden, den Einsatz von teurem und schwerem Holz dadurch zu vermeiden, daß der leichte Holzkern an seiner Anschlußfläche im Bereich des dorsalen Anschlags verstärkt worden ist. Eine aufgesetzte Metallplatte führt jedoch zu einer unerwünschten gleitenden Bewegung des Adapters auf der Anschlußfläche. Es ist ferner versucht worden, die Oberfläche des Holzkerns an der Stelle des dorsalen Anschlags durch einen Hartholzeinsatz zu ersetzen. Es hat sich jedoch gezeigt, daß eine stabile Verbindung des Hartholzeinsatzes mit dem Holzkern durch Verklebung o. ä. aufgrund der starken Belastung nicht möglich ist.

Der Erfindung liegt die Aufgabe zugrunde, einen künstlichen gelenklosen Fuß der eingangs erwähnten Art so auszubilden, daß eine Abnutzung des Holzkerns im Bereich des dorsalen Anschlags – auch bei der Verwendung von einem relativ weichen und leichten Holz für den Holzkern– wirksam vermieden wird.

Diese Aufgabe wird erfindungsgemäß dadurch gelöst, daß der Holzkern im Bereich des dorsalen Anschlags eine nach oben offene Ausnehmung aufweist, die mit einem duroplastischen Gießharz gefüllt ist, dessen Oberfläche mit der Anschlußfläche fluchtet.

In einer bevorzugten Ausführungsform ist das duroplastische Gießharz mit einem Füllstoff versehen.

In überraschend einfacher Weise läßt sich die Abnutzung des Holzkerns im Bereich des dorsalen Anschlags dadurch vermeiden, daß in die Ausnehmung des Holzkerns duroplastisches Gießharz eingefüllt wird. Das duroplastische Gießharz stellt eine sichere feste Verbindung mit dem Holzkern her und läßt sich in einfacher Weise so einfüllen, daß seine Oberfläche mit der Anschlußfläche exakt fluchtet. Gegebenenfalls kann hierzu eine anschließende gemeinsame Bearbeitung der Anschlußfläche vorgenommen werden.

Versuche haben gezeigt, daß trotz extremer Belastung der Oberfläche des Gießharzes dieses fest in dem Holzkern verankert ist und keine Abnutzungserscheinungen zeigt.

Der erfindungsgemäße künstliche gelenklose Fuß kann daher aufgrund der geschilderten einfachen und preiswerten Maßnahme mit einem preiswerten, leichten Holzkern aufgebaut werden.

Als duroplastisches Gießharz wird vorzugsweise ein gefülltes Gießharz verwendet, wobei als Füllstoff Schiefermehl, Glas, Sand o. dgl. in Frage kommt. Durch den Füllstoff wird nicht nur der Preis für das verwendete Gießharz gesenkt, sondern auch die beim Aushärten des Gießharzes regelmäßig auftretende exotherme Reaktion gemindert und die Druckfestigkeit erhöht.

Als aushärtendes Gießharz wird regelmäßig ein Zwei-Komponenten-Gießharz, wovon eine Komponente als Härter wirkt, verwendet werden. Es ist aber auch möglich, ein Gießharz zu verwenden, daß unter Einwirkung von ultravioletter Strahlung aushärtet.

Die Erfindung soll im folgenden anhand eines in der Zeichnung dargestellten Ausführungsbeispiels näher erläutert werden. Es zeigen:

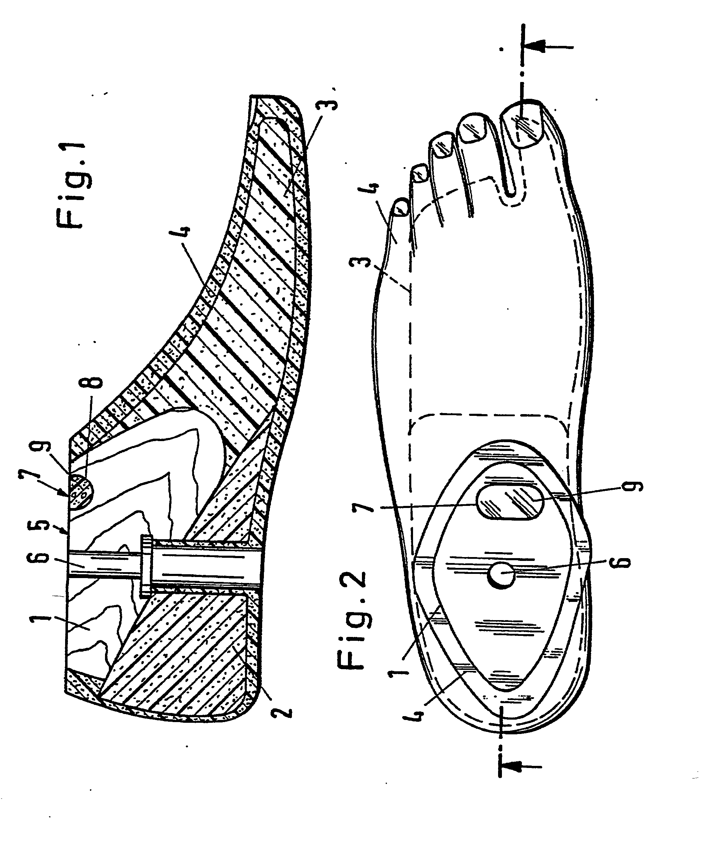

Figur 1 - einen Längsschnitt durch einen künstlichen gelenklosen Fuß

Figur 2 - eine Draufsicht auf den Fuß gemäß Figur 1.

Der in der Zeichnung dargestellte Fuß besteht im wesentlichen aus einem Holzkern 1, einem im Fersenbereich des Fußes angeordneten Fersenkeil 2, einem an die Vorderseite des Holzkerns 1 und des Fersenkeils 2 angeschlossenen Innenfuß 3 sowie einer hautbildenden Umhüllungsschicht 4. Der Kern 1 bildet eine obere Anschlußfläche 5, auf die ein Adapter zur Verbindung mit einem künstlichen Bein aufgesetzt wird. Zur Befestigung des Adapters dient eine Bohrung 6, die sich senkrecht zur Anschlußfläche 5 in den Holzkern 1 erstreckt und sich am unteren Rand des Holzkerns 1 stufenförmig er-

weitert. Die Bohrung 6 dient zur Aufnahme des Schaftes und des Kopfes einer Befestigungsschraube.

Der (nicht dargestellte) Adapter liegt mit seiner vorderen Kante an einer Stelle der Anschlußfläche 5 auf, die einen dorsalen Anschlag 7 bildet. Im Bereich des dorsalen Anschlags 7 ist der Holzkern 1 an der Oberfläche ausgehöhlt und bildet eine nach oben offene, langlochförmige Ausnehmung 8.

In die Ausnehmung 8 ist ein duroplastisches Gießharz 9 eingefüllt, das mit einem Schiefermehl, mit Glasstaub oder Sand als Füllstoff versetzt ist. Die Oberfläche des Gießharzes 9 fluchtet mit der Anschlußfläche 5, so daß eine gemeinsame ebene Oberfläche entsteht.

Das Gießharz dringt in die Holzporen der Ausnehmung 8 ein und verbindet sich fest mit dem Holzkern 1. Die Oberfläche des Gießharzes 9 wird durch die Belastung des Adapters nicht beschädigt. Die Festigkeit der Verbindung des Gießharzes 9 mit dem Holzkern 1 wird durch die Belastung nicht beeinträchtigt.

Unabhängig von den Eigenschaften des Holzkerns 1 ist der dorsale Anschlag 7 daher für jede Belastung durch den Adapter geeignet.

## Patentansprüche

1. Künstlicher gelenkloser Fuß mit einem eine obere Anschlußfläche (5) bildenden Holzkern (1) und mit elastischen, mit dem Holzkern (1) verbundenen Kunststoffmaterialien (2, 3, 4) zur Ausbildung der Fußform und Gewährleistung eines flexiblen Abrollvorganges, wobei der Holzkern (1) auf seiner Anschlußfläche (5) einen dorsalen Anschlag (7) für einen mit ihm verbindbaren Adapter bildet, dadurch gekennzeichnet, daß der Holzkern (1) im Bereich des dorsalen Anschlags (7) eine nach oben offene Ausnehmung (8) aufweist, die mit einem duroplastischen Gießharz (9) gefüllt ist, dessen Oberfläche mit der Anschlußfläche (5) fluchtet.

2. Künstlicher Fuß nach Anspruch 1, dadurch gekennzeichnet, daß das duroplastische Gießharz mit einem Füllstoff versehen ist.

## Claims

1. Artificial jointless foot with a wooden core (1) forming an upper contact surface (5), and with elastic synthetic materials (2, 3, 4) connected to the wooden core (1) for the purpose of forming the foot shape and ensuring a flexible rolling action, the wooden core (1) forming on its contact surface (5) a dorsal stop (7) for an adapter which can be connected to it, characterized in that the wooden core (1) has, in the area of the dorsal stop (7), a recess (8) which is open towards the top and which is filled with a duroplastic casting resin (9) whose upper surface is flush with the contact surface (5).

2. Artificial foot according to Claim 1, characterized in that the duroplastic casting resin is provided with a filler.

## Revendications

1. Pied artificiel non articulé comportant un noyau en bois (1) formant une face de raccordement supérieure (5) et des matières plastiques (2, 3, 4) élastiques assujetties au noyau en bois (1) pour constituer la forme du pied et garantir un processus de flexion souple, le noyau en bois (1) formant sur sa face de raccordement (5) une butée dorsale (7) pour un raccord pouvant y être relié, caractérisé en ce que le noyau en bois (1) dans la zone de la butée dorsale (7) présente un évidement (8) ouvert vers le haut qui est rempli d'une résine moulée thermodurcissable (9), dont la surface est disposée au ras de la face de raccordement (5).

2. Pied artificiel suivant la revendication 1, caractérisé en ce que la résine moulée thermodurcissable est pourvu d'une charge.

Fig.1

Fig.2

EP 0 275 552 B1